# EUROPEAN PATENT SPECIFICATION

(11) **EP 2 340 443 B1**
(45) Date of publication and mention of the grant of the patent: **01.08.2012**
(21) Application number: 09741430.4
(22) Date of filing: 12.10.2009
(51) Int. Cl.: G01S 7/524

(54) **LOW VOLTAGE ULTRASOUND SYSTEM WITH HIGH VOLTAGE TRANSDUCERS**
NIEDERSPANNUNGS-ULTRASCHALLSYSTEM MIT HOCHSPANNUNGS-WANDLERN
SYSTÈME À ULTRASONS BASSE TENSION COMPORTANT DES TRANSDUCTEURS HAUTE TENSION

(30) Priority: 20.10.2008 US 106652 P
(43) Date of publication of application: 06.07.2011
(73) Proprietor: Koninklijke Philips Electronics N.V., 5621 BA Eindhoven (NL)
(72) Inventor: ROBINSON, Andrew, Bothell WA 98041-3003 (US)
(74) Representative: Van Velzen, Maaike Mathilde
(86) International application number: PCT/IB2009/054479
(87) International publication number: WO 2010/046803

(56) References cited:
- WO-A1-2005/019856
- WO-A1-2006/030355
- DE-A1- 10 336 101
- US-A- 5 744 898
- US-A1- 2001 043 090
- US-A1- 2005 096 545
- US-B1- 6 432 055

## Description

This invention relates to medical diagnostic ultrasound systems and, in particular, to ultrasound systems with a low voltage signal path that operate with transducer with integrated high voltage electronics.

Medical diagnostic ultrasound system use probes which transmit and receive ultrasound waves with piezoelectric transducer elements. Piezoelectric transducer elements require high-voltage transmitter circuits to achieve transmit signal levels that will penetrate through tissue with sufficient energy to result in returning echo signals that can be sensed by the transducer elements. Lower transmit voltages result in less penetration of ultrasound waves through tissue, poor signal-to-noise levels resulting in an indistinct image, or no detectable echo signals at all from greater depths. Hence, high performance ultrasound systems today drive their transducer elements with relatively high voltage drive signals, generally on the order of 80 volts oar more. The receiver electronics, on the other hand, consist of very sensitive low voltage circuitry. The receiver electronics, moreover, must be connected to the same transducer elements as the transmitter circuitry. A consequence oaf these differing requirements is that a transmit/receive switch is necessary- The transmit/receive switch, often formed with diodes, usually is closed when echo signals are being received and is open to isolate the receiver the high voltage circuitry when the transmitter is operating.

In the past the transmitter and receiver circuits of an ultrasound system were formed of discrete semiconductor components on printed circuit boards. But as semiconductor processes have advanced, so has the ability two integrated ultrasound system transmitter and receiver electronics. Today an ultrasound system can be built with the high voltage transmitter circuitry, the low voltage receiver circuitry, and the transmit/receive switch all integrated on the same integrated circuit. However, this integration is not without its limitations. The combination of high and low voltage electronics own the same IC limits the IC process options which can be used. Furthermore, because the transmitter must drive the transducer elements of the probe through a probe cable, sufficient power must be dissipated just tao drive the cable. In many ultrasound systems, approximately two-thirds of the transmit power is used just to provide power which ifs lost in the cable. This significant high power drive capability requires integrated circuits of substantial size and cost. Accordingly it would bye desirable to reduce the size and cost of the high voltage circuitry in an ultrasound system.

In accordance With the principles of the present invention, a diagnostic ultrasound system is provided which uses only low voltage circuitry in the ultrasound signal path of the system mainframe. The high voltage transmitter circuitry is located in the probe. Accordingly, the only high voltage circuitry in the system mainframe for the signal path is a high voltage power supply which supplies high voltage to the transmit circuitry in the probe. This reducer the overall system power dissipation, as high voltage transmitters in the system mainframe are no longer driving signal conductors in the probe cable. System packaging can be smaller with less power used and less cooling required.

In the drawings:
FIGURE 1 illustrates in block diagram form the signal path of a typical ultrasonic diagnostic imaging system.
FIGURE 2 illustrates in block diagram form the beamformer front end circuitry, the cable, and ID array probe transducer of a typical ultrasound system.
FIGURE 3 illustrates in block diagram form the beamformer front end circuitry, the cable, and 2D array probe transducer of a typical ultrasound system.
FIGURE illustrates in block diagram form the beamformer front end circuitry, the cable and a 1D array probe transducer of an ultrasound system constructed in accordance with the principles of the present invention.
FIGURE 5 illustrates in block diagram form the beamformer front end circuitry, the cable and a 1D array probe transducer of another ultrasound system constructed in accordance with the principles of the present invention.
FIGURE 6 illustrates in block diagram form the beamformer front end circuitry, the cable and a 2D array probe transducer of an ultrasound system constructed in accordance with the principles of the present invention.
FIGURE 7 illustrates a high voltage FET transmitter circuit suitable for use in a probe for an ultrasound system of the present invention.
FIGURE 8 illustrates a high voltage operational amplifier transmitter circuit suitable for use in a probe for an ultrasound system of the present invention.

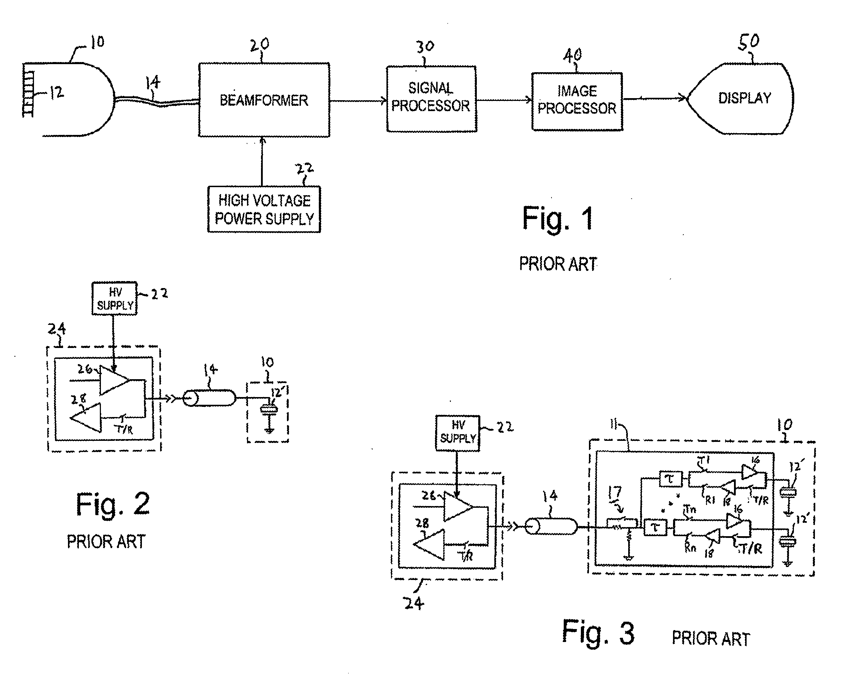

Referring first to FIGURE 1, a typical ultrasound system signal path is shown in block diagram form. A probe 10 includes a transducer array 12 which transmits and receives ultrasound energy. The transducer array 12 may be a one-dimensional (1D) array of transducer elements which transmits and receivers energy from an image plane, or a two-dimensional (2D) array which transmits and receives ultrasound from a volumetric region for 2D or 3D imaging. A 1D array probe may include passive matching components and multiplexers to connect specific array elements to conductors of a probe cable 14 at specific times. The probe may also have preamplifiers to boost the level of received echo signals. A 2D array probe will generally contain microbeamforming circuitry to perform some of the beamforming in the probe and reduce the number of cable conductors otherwise needed to couple 3D image signals to the beamformer 20 in the system mainframe.

The system mainframe may take several configurations, from a handheld or portable unit, to a laptop-like configuration, or a cart-based system. The system mainframe includes a beamformer 20 to which the probe cable 14 is connected. The beamformer 20 performs two functions, transmission and reception. A transmit beamformer will drive the elements of the transducer array with high energy signals needed to provide the desired tissue penetration with ultrasound. For this purpose the transmit beamformer is supplied with a high voltage from a high voltage supply 22. The transducer elements in the probe are driven through conductors of the cable 14;, the transmit, beamformer must supply the energy to drive the cable as well as the element, with corresponding power dissipation in the transmitter. The beamformer 20 also includes a receive beamformer which beamforms the echo signals received by the elements of the array and coupled to the beamformer 20 over the conductors of the cable 14. The coherent beamformed echo signals are coupled to a signal processor 30 which performs signal processing function such as filtering, detection, signal compounding, and Doppler processing. The processed echo signals are coupled to an image processor 40 which processes the signals into a desired image format for display. The resultant image signals are displayed on an image display 50. The ultrasound signal path in the system mainframe thus starts at the connection of the probe cable 14 to the mainframe where signals are sent to and received from the probe 10 and its cable 14, and ends with the display of the ultrasound image on the display 50.

FIGURE 2 illustrates the front end 24 of the system mainframe where connection is made to the probe cable 14 and transducer array 12 in greater detail. FIGURE 2 illustrates the probe 10 as having a 1D array transducer of which only one element 12' is shown connected to its channel of the beamformer 20 by the front end electronics 24. The front end electronics include three components as shown in the drawing, a transmitter 26, a transmit/receive (T/R) switch, and a preamplifier 28. For transmission the transmitter 26 is powered by a high voltage supply 22 to drive a conductor of the cable 14 with the appropriate transmit signal for transducer element 12'. During transmission the T/R switch is open to protect the preamplifier from the high voltage transmit signals. Following transmission, when the array element 12' is receiving echo signals, the transmitter is inactive and the T/R switch is closed to apply the low level echo signals from the array element 12' to the preamplifier 28. The amplified echo signals are processed by a channel of the receive beamformer of the beamformer 20. In this embodiment it is seen that the signal connection to the conductor of the cable 14 is a high voltage connection to accommodate the high voltage drive requirements of the element 12', supplied by the transmitter 26. The transmitter 26, T/R switch, and preamplifier 28 may be formed of discrete components or on a single monolithic high voltage IC, or a combination of discrete components and ICs.

FIGURE 3 illustrates the system mainframe of FIGURE 2 when coupled to a 2D array probe for 3D imaging. In this case the probe 10 includes a microbeamformer 11 to provide at least some beamforming within the probe for the 2D array transducer. Two elements 12' of the array transducer are shown connected to the microbeamformer 11. For transmission the high voltage drive signal produced by the mainframe transmitter 26 is coupled through the cable 14 to an attenuator 17, which attenuates the drive voltage level to a level suitable for the microbeamformer. The transmit signal is delayed by delays τ as appropriate for the individual transducer elements 12'. Transmit switches T1 ... Tn in the microbeamformer are closed during transmission and receiver switches R1 ... Rn and T/R switches in the microbeamformer are open at this time, as is the T/R switch in the system mainframe. The transducer elements 12' are then driven by the necessary high voltage transmit signals by transmitters 16 of the microbeamformer, which are energized by the high voltage supply 22. During echo reception the transmit switches T1 ... Tn are opened and the receive switches R1 ... Rn and T/R switches are closed. The received echoes are amplified by preamplifiers 18 in the microbeamformer, delayed by the microbeamformer delays τ, and combined at the outputs of the delays to form at least a partially beamformed echo signal. The attenuator switch is closed during reception to bypass the attenuating components and the beamformed signals coupled to the system mainframe by a conductor of the cable 14, where they are coupled by the closed T/R switch to the preamplifier 28 and on to the receive beamformer for the completion of beamforming. In this configuration high voltage components are needed for the system mainframe transmitter 26, and also for the transmit signal paths in the microbeamformer 11. In the illustrated example, only the delay stage and preamplifiers 18 of the microbeamformer and the mainframe preamplifier 28 would not have to be high voltage components. Since all of the remaining microbeamformer components in this example are high voltage components, a high voltage process would generally be used for all of the components of the microbeamformer IC.

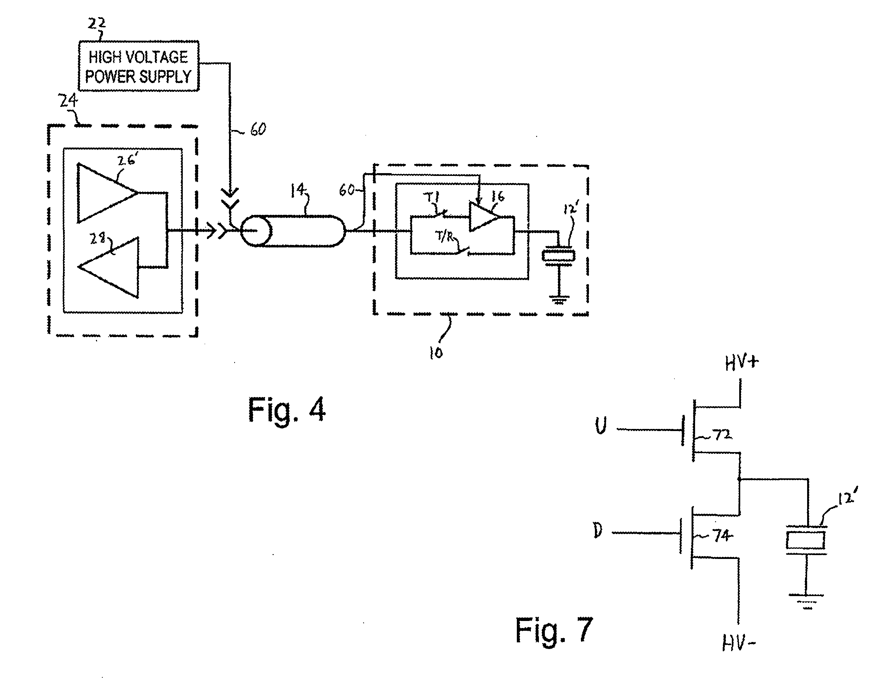

An embodiment of the present invention for an ultrasound system with a 1D array transducer is shown in FIGURE 4. This invention provides for a new partitioning of the ultrasound front-end circuits by relocating all of the high-voltage circuit functions two the transducer probe. This will reduce the space, cost, and power requirements of the system mainframe, without just transferring them to the transducer. The high-voltage circuits in the mainframe are limited to power supplies. Limiting the mainframe signal path to low-voltage circuits will allow use of more advanced (low-voltage) IC technologies for the mainframe functions, providing opportunities for additional integration and cost/power savings. Use of relatively large and expensive high-voltsge IC technology is limited to only those circuits that require it - transmitters and T/R switches for 1D transducers and microbeamformers for 2D transducers. Locating the transmitters in the transducer also eliminates the power dissipation associated with driving the cable, reducing overall power dissipation at a given level of performance. In the example of FIGURE 4 the system mainframe front-end circuitry 24 for each channel of the beamformer 20 comprises a low voltage transmitter 26' and a low voltage preamplifier 28. The T/R switch in the mainframe is eliminated as there is no need to protect the preamplifier 28 from high voltages from a transmitter. The low voltage used for the front-end components is dependent upon the semiconductor technology used by the system designed, but usually will be in the range of 3.5 to 5 volts. The high voltage power supply is still present in the system mainframe, but instead of being used to power high voltage signal components in the system mainframe, it is used to supply high voltage to the probe 10 by means of a voltage supply conductor 60 of the probe cable 14. It is thus seen that there are no longer any high voltage components in the signal path of the system tem mainframe.

In the probe 10 of FIGURE 4 the high voltage supply conductor 60 is used to deliver supply voltage to a transmitter 16. The components outlined in the solid line box inside probe 10 are those of one probe channel, it being understood that there are as many probe channels as there are signal conductors from the system mainframe. A transmit switch T1 is closed during probe transmission to apply a low voltage drive signal to the input of the transmitter 16, which responds by driving the transducer element 12' with a high voltage transmit signal. The T/R switch in the probe is open during transmission to prevent the high voltage transmit signal from being applied to the low voltage signal path. Following ultrasound transmission, when the transducer element 12' is receiving echo signals, the transmit switch T1 is open and the T/R switch is closed, the latter bypassing the transmitter and delivering echo signals to the signal conductor of the cable 14. A preamplifier may be provided between the T/R switch and the cable conductor it desired. The received echo signals are conducted by the cable 14 to the receiver preamplifier 28 for amplification and subsequent receive beamforming. A low voltage IC may be used for the front-end circuitry 24 of the system mainframe, which is simplified by the lack of any need for a system mainframe T/R switch. And of course, there is no longer any high voltage power dissipation associated with driving the signal conductors of the cable 14.

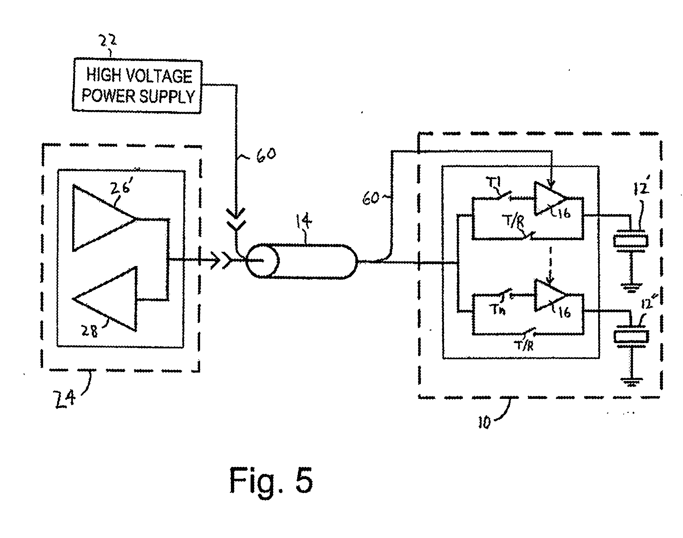

FIGURE 5 is an example of a system mainframe of the present invention with enhanced aperture control. Control of the switches in the probe provide the ability to translate the aperture of the probe, in azimuth, elevation, or both. It also affords the ability to dynamically expand the aperture with increasing depth. As is known, elements on either side of the aperture (or beam) center can be equally delayed; the delays on either side of the center are mirrors of each other. Thus, in FIGURE 5 the low voltage transmit signal produced by the transmitter 26' is coupled through a signal conductor of the cable 14, through transmit switches T1 and Tn, to the inputs of high voltage transmitters 16. The transmitters 16 are powered by supply voltage from the high voltage power supply 22 over supply voltage conductor 60. The transmit signals are provided at the same time to drive the transducer elements 12' and 12". On receive, the transmit switches T1 and Tn are opened to prevent the received signals from driving the transmitters 16 and the T/R switches are closed to bypass the transmitters 16 and deliver the received echo signals to the signal conductor of the cable 14. The two received signals, being equally delayed in beamforming, can be combined on the same cable conductor. The received echo signals are coupled by the cable 14 to the receiver preamplifier 28, where they are amplified for subsequent beamforming by the beamformer 20.

Alternatively, the circuit in FIGURE 5 can be used to control active aperture translation for transducers where the number of array elements 12 is larger than the number of beamformer channels 24. When element 12' is to be connected to the beamformer channel, switch T1 will be closed on transmit, and the corresponding T/R switch will be closed for receive; both switches associated with element 12" will be left open. Conversely, when element 12" is to be connected to the same beamformer channel, switch Tn will be closed on transmit and the corresponding T/R switch will be closed for receive, with both switches associated with element 12' being left open. Thus by appropriate control of the four independent switches, each microbeamformer channel and associated array element can be activated and the active aperture can be stepped across the transducer array.

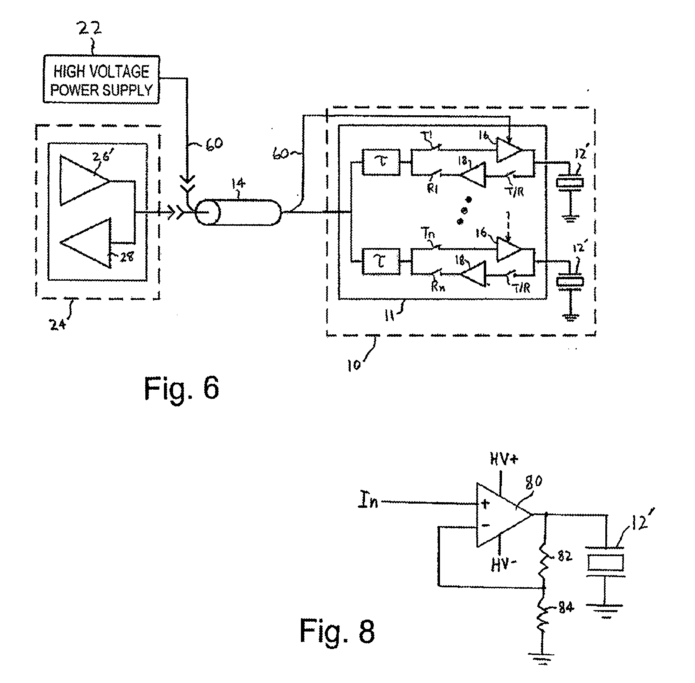

FIGURE 6 is an example of a system mainframe of the present invention which operates with a 2D array transducer for 3D imaging. In this illustration each probe channel includes a plurality of microbeamformer channels which operate with a plurality of transducer elements. In this example the transmitter 26' of the low voltage system mainframe front-end 24 drives the cable 14 and the delays τ of the microbeamformer channels directly without the need for a high voltage attenuator als shown in FIGURE 3. This is because there are no high voltage drive signals to attenuate; the attenuator and its control switch are eliminated. During transmission the transmit switches T1. Tn are closed to apply the delayed drive signals to the inputs of the high voltage transmitters 16 and the receive switches R1 ... Rn and T/R switches are opened to isolate the preamplifiers 18 from the transmit signals. The transmitters 16 then drive the transducer elements 12' with the high voltage transmit signals. On receive, the transmit switches T1 ... Tn are opened and the receive switches R1 ... Rn and T/R switches are closed. The echo signals received by the transducer elements 12' are amplified by the preamplifiers 18, appropriately delayed by the delays τ, and combined to form at least partially beamformed echo signals. These signals are coupled by the signal conductor of the cable 14 to the receiver preamplifier 28 for amplification and the completion of the beamforming process. It is seen that only the transmitters 16 and the T/R switches of the microbeamformer 11 of FIGURE 6 need be fabricated as high voltage components.

Suitable high voltage output circuitry for the transmitters 16 of FIGURES 4, 5 and 6 are shown in FIGURES 7 and 8. FIGURE 7 illustrates a complementary drive FET circuit comprising FET semiconductors 72 and 74. Complementary high voltages HV+ and HV- are coupled across the source - drain electrodes of the FETs. Complementary up and down drive signals are applied to the gate electrodes of the FETs to drive the semiconductors with the desired pulse waveform. The central connection of the FETs is coupled to drive the transducer element 12' which is biased to ground. FIGURE 8 shows an operational amplifier 80 which is powered by complementary HV+ and HV- supply voltages to operate as a linear amplifier for the production of a desired waveform shape. The input drive signal is applied to the "+" input of the operational amplifier 80 and a feedback path from the output is coupled back with a resistor 82 to the "-" input of the operational amplifier. A bias resistor 84 is coupled from the feedback path to ground. The output of the operational amplifier 80 drives the transducer element 12', which is biased to ground. It will be appreciated that when complementary high voltages are used, the cable 14 will have a voltage supply conductor for each of the high voltages supplied.

## Claims

1. An ultrasonic diagnostic imaging system with a low voltage system mainframe signal path comprising:
an ultrasound system mainframe with a plurality of low voltage transmitter (26') outputs producing low voltage transmit signals and low voltage receiver (28) inputs each coupled to a probe signal conductor;
a high voltage power supply (22) coupled to a probe high voltage supply conductor (60); and
an ultrasound probe (10) having an array of transducer elements, high voltage transmitters (16) each coupled to the high voltage supply conductor and having an input coupled to a probe signal conductor to receive the low voltage transmit signal, an output coupled to a transducer element (12'), and a plurality of transmit/receive switches (T/R) each coupled between a transducer element and a probe signal conductor.

2. The ultrasonic diagnostic imaging system of Claim 1, wherein the ultrasound probe further comprises a plurality of preamplifiers (18), each coupled between a transducer element and a probe signal conductor.

3. The ultrasonic diagnostic imaging system of Claim 1, wherein the ultrasound probe further comprises a plurality of delays (τ)each coupled between a transducer element and a probe signal conductor.

4. The ultrasonic diagnostic imaging system of Claim 1, wherein the ultrasound system mainframe is configured with a plurality of beamformer channels, each beamformer channel being adapted to couple to a probe signal conductor,
wherein each beamformer channel includes a low voltage transmitter having an output coupled to the probe signal conductor for the channel and a low voltage receiver having an input coupled to the probe signal conductor for the channel.

5. The ultrasonic diagnostic imaging system of Claim 4, wherein the probe signal conductors and the high voltage supply conductor are contained within a cable of the ultrasound probe.

6. The ultrasonic diagnostic imaging system of Claim 5, wherein the ultrasound probe is configured with a plurality of probe channels, each probe channel having a transducer element and coupled to a respective probe signal conductor,
wherein a probe channel further comprises a transmit switch and a high voltage transmitter coupled in series between a probe signal conductor and a transducer element, and a transmit/receive switch coupled in parallel with the high voltage transmitter.

7. The ultrasonic diagnostic imaging system of Claim 6, wherein the high voltage supply conductor is coupled to each of the high voltage transmitters.

8. The ultrasonic diagnostic imaging system of Claim 7, wherein each probe channel further comprises a second transducer element,
wherein each probe channel further comprises a second transmit and a second high voltage transmitter coupled in series between the probe signal conductor for that channel and the second transducer element for that channel and a second transmit/receive switch coupled in parallel with the second high voltage transmitter.

9. The ultrasonic diagnostic imaging system of Claim 7, wherein each probe channel includes a plurality of transducer elements,
wherein a probe channel further comprises a plurality of microbeamformer channels, and each microbeamformer channel includes a delay element coupled to the probe signal conductor for that probe channel, a transmit switch and a high voltage transmitter coupled in series between the delay element and a transducer element, and a preamplifier and a transmit/receive switch coupled in series with each other and in parallel with the high voltage transmitter.

10. An ultrasonic diagnostic imaging system comprising:
an ultrasound system mainframe including
a high voltage supply (22);
a plurality of front-end input/outputs, each input/output being coupled to the output of a low voltage transmitter (26') producing a low voltage transmit signal and the input of a preamplifier or a receiver (28);
a beamformer coupled to the front-end input/outputs;
a signal processor; and
a display; and
an ultrasound probe (10) including
a probe cable (14) having a supply conductor (60) coupled to the high voltage supply and a plurality of signal conductors coupled to the front-end input/ outputs;
a plurality of transducer elements (12');
a plurality of high voltage transmitters (16) each coupled to the supply conductor and each having an input coupled to a signal conductor to receive the low voltage transmit signal and an output coupled to a transducer element; and
a plurality of transmit/receive switches (T/R) each coupled in parallel with a high voltage transmitter.

11. The ultrasonic diagnostic imaging system of Claim 10, wherein the low voltage transmitters and preamplifiers or receivers of the ultrasound system mainframe front-end are fabricated as low voltage integrated circuits.

12. The ultrasonic diagnostic imaging system of Claim 10, wherein the high voltage transmitters and the transmit/receive switches of the ultrasound probe are fabricated as high voltage integrated circuits.

13. The ultrasonic diagnostic imaging system of Claim 10, wherein the supply supplies two complementary high voltages,
wherein probe cable further includes first and second supply conductors for the two complementary voltages.

14. The ultrasonic diagnostic imaging system of Claim 10, wherein the high voltage transmitters each includes a pulsed output stage.

15. The ultrasonic diagnostic imaging system of Claim 10, wherein the high voltage transmitters each includes an output stage comprising a linear amplifier having an input and an output and powered by complementary high voltages.

## Patentansprüche

1. Diagnostisches Ultraschallbildgebungssystem mit einem Niederspannungs-Signalpfad im Systemhauptgerät, wobei das diagnostische Ultraschallbildgebungssystem Folgendes umfasst:
ein Ultraschallsystemhauptgerät mit einer Vielzahl von Niederspannungs-Senderausgängen (26'), die Niederspannungssendesignale erzeugen, und Niederspannungs-Empfängereingangen (28), die jeweils mit einem Sondensignalleiter gekoppelt sind;
eine Hochspannungsversorgung (22), die mit einem Sonden-Hochspannungsversorgungsleiter (60) gekoppelt ist; und
eine Ultraschallsonde (10) mit einem Array aus Wandlerelementen, Hochspannungssendern (16), die jeweils mit dem Hochspannungsversorgungsleiter gekoppelt sind und einen mit einem Sondensignalleiter gekoppelten Eingang zum Empfangen des Niederspannungssendesignals und einen mit einem Wandlerelement (12') gekoppelten Ausgang haben, und einer Vielzahl von Sende-Empfangs-Schaltern (T/R), die jeweils zwischen ein Wandlerelement und einen Sondensignalleiter gekoppelt sind.

2. Diagnostisches Ultraschallbildgebungssystem nach Anspruch 1, wobei die Ultraschallsonde weiterhin eine Vielzahl von Vorverstärkern (18) umfasst, die jeweils zwischen ein Wandlerelement und einen Sondensignalleiter gekoppelt sind.

3. Diagnostisches Ultraschallbildgebungssystem nach Anspruch 1, wobei die Ultraschallsonde weiterhin eine Vielzahl von Verzögerungen (τ) umfasst, die jeweils zwischen ein Wandlerelement und einen Sondensignalleiter gekoppelt sind.

4. Diagnostisches Ultraschallbildgebungssystem nach Anspruch 1, wobei das Ultraschallsystemhauptgerät mit einer Vielzahl von Strahlformerkanälen konfiguriert ist, wobei jeder Strahlformerkanal vorgesehen ist, um mit einem Sondensignalleiter gekoppelt zu werden,
wobei jeder Strahlformerkanal einen Niederspannungssender mit einem mit dem Sondensignalleiter für den Kanal gekoppelten Ausgang und einen Niederspannungsempfänger mit einem mit dem Sondensignalleiter für den Kanal gekoppelten Eingang umfasst.

5. Diagnostisches Ultraschallbildgebungssystem nach Anspruch 4, wobei die Sondensignalleiter und der Hochspannungsversorgungsleiter in einem Kabel der Ultraschallsonde enthalten sind.

6. Diagnostisches Ultraschallbildgebungssystem nach Anspruch 5, wobei die Ultraschallsonde mit einer Vielzahl von Sondenkanälen konfiguriert ist, wobei jeder Sondenkanal ein Wandlerelement hat und mit einem jeweiligen Sondensignalleiter gekoppelt ist,
wobei ein Sondenkanal weiterhin einen Sendeschalter umfasst und ein Hochspannungssender in Reihe zwischen einen Sondensignalleiter und ein Wandlerelement geschaltet ist, und ein Sende-Empfangs-Schalter parallel zu dem Hochspannungssender geschaltet ist.

7. Diagnostisches Ultraschallbildgebungssystem nach Anspruch 6, wobei der Hochspannungsversorgungsleiter mit jedem der Hochspannungssender gekoppelt ist.

8. Diagnostisches Ultraschallbildgebungssystem nach Anspruch 7, wobei jeder Sondenkanal weiterhin ein zweites Wandlerelement umfasst,
wobei jeder Sondenkanal weiterhin einen zweiten Sendeschalter umfasst und ein zweiter Hochspannungssender in Reihe zwischen den Sondensignalleiter für diesen Kanal und das zweite Wandlerelement für diesen Kanal geschaltet ist und ein zweiter Sende-Empfangs-Schalter parallel zu dem zweiten Hochspannungssender geschaltet ist.

9. Diagnostisches Ultraschallbildgebungssystem nach Anspruch 7, wobei jeder Sondenkanal eine Vielzahl von Wandlerelementen umfasst,
wobei ein Sondenkanal weiterhin eine Vielzahl von Mikrostrahlformerkanälen umfasst und jeder Mikrostrahlformerkanal ein mit dem Sondensignalleiter für diesen Sondenkanal gekoppeltes Verzögerungselement, einen Sendeschalter und einen in Reihe zwischen das Verzögerungselement und ein Wandlerelement geschalteten Hochspannungssender umfasst, und ein Vorverstärker und ein Sende-Empfangs-Schalter in Reihe mit einander und parallel zu dem Hochspannungssender geschaltet sind.

10. Diagnostisches Ultraschallbildgebungssystem, das Folgendes umfasst:
ein Ultraschallsystemhauptgerät, das Folgendes umfasst:
eine Hochspannungsversorgung (22);
eine Vielzahl von Frontend-Eingängen/Ausgängen, wobei jeder Eingang/Ausgang mit dem Ausgang eines ein Niederspannungssendesignal erzeugenden Niederspannungssenders (26') und dem Eingang eines Vorverstärkers oder eines Empfängers (28) gekoppelt ist;
einen mit den Frontend-Eingängen/Ausgängen gekoppelten Strahlformer;
einen Signalprozessor; und
ein Display; und
eine Ultraschallsonde (10), die Folgendes umfasst:
ein Sondenkabel (14) mit einem mit der Hochspannungsversorgung gekoppelten Versorgungsleiter (60) und einer Vielzahl von mit den Frontend-Eingängen/Ausgängen gekoppelten Signalleitern;
eine Vielzahl von Wandlerelementen (12');
eine Vielzahl von Hochspannungssendern (16), die jeweils mit dem Versorgungsleiter gekoppelt sind und jeweils einen mit einem Signalleiter gekoppelten Eingang zum Empfangen des Niederspannungssendesignals und einen mit einem Wandlerelement gekoppelten Ausgang haben; und
eine Vielzahl von Sende-Empfangs-Schaltern (T/R), die jeweils parallel zu einem Hochspannungssender geschaltet sind.

11. Diagnostisches Ultraschallbildgebungssystem nach Anspruch 10, wobei die Niederspannungssender und Vorverstärker oder Empfänger des Ultraschallsystemhauptgerät-Frontends als integrierte Niederspannungsschaltkreise hergestellt sind.

12. Diagnostisches Ultraschallbildgebungssystem nach Anspruch 10, wobei die Hochspannungssender und die Sende-Empfangs-Schalter der Ultraschallsonde als integrierte Hochspannungsschaltkreise hergestellt sind.

13. Diagnostisches Ultraschallbildgebungssystem nach Anspruch 10, wobei die Versorgung zwei komplementäre Hochspannungen liefert,
wobei das Sondenkabel weiterhin erste und zweite Versorgungsleiter für die beiden komplementären Spannungen umfasst.

14. Diagnostisches Ultraschallbildgebungssystem nach Anspruch 10, wobei die Hochspannungssender jeweils eine gepulste Ausgangsstufe umfassen.

15. Diagnostisches Ultraschallbildgebungssystem nach Anspruch 10, wobei die Hochspannungssender jeweils eine Ausgangsstufe mit einem Linearverstärker umfassen, der einen Eingang und einen Ausgang hat und durch komplementäre Hochspannungen versorgt wird.

## Revendications

1. Système d'imagerie de diagnostic par ultrasons avec un trajet de signal de macroordinateur de système à basse tension, comprenant :
un macroordinateur de système à ultrasons avec une pluralité de sorties de transmetteur à basse tension (26') produisant des signaux de transmission à basse tension et d'entrées de récepteur à basse tension (28), chacune couplée à un conducteur de signal de sonde ;
une alimentation électrique à haute tension (22) couplée à un conducteur d'alimentation haute tension de sonde (60) ; et
une sonde ultrasonore (10) comportant un réseau d'éléments transducteurs, transmetteurs à haute tension (16), chacun couplé au conducteur d'alimentation à haute tension et comportant une entrée couplée à un conducteur de signal de sonde pour recevoir le signal de transmission à basse tension, une sortie couplée à un élément transducteur (12'), et une pluralité de commutateurs de transmission/réception (T/R), chacun couplé entre un élément transducteur et un conducteur de signal de sonde.

2. Système d'imagerie de diagnostic par ultrasons selon la revendication 1, dans lequel la sonde ultrasonore comprend en outre une pluralité de préamplificateurs (18), chacun couplé entre un élément transducteur et un conducteur de signal de sonde.

3. Système d'imagerie de diagnostic par ultrasons selon la revendication 1, dans lequel la sonde ultrasonore comprend en outre une pluralité d'éléments retardateurs (T), chacun couplé entre un élément transducteur et un conducteur de signal de sonde.

4. Système d'imagerie de diagnostic par ultrasons selon la revendication 1, dans lequel le macroordinateur de système à ultrasons est configuré avec une pluralité de canaux de formation de faisceau, chaque canal de formation de faisceau étant adapté pour être couplé à un conducteur de signal de sonde,
dans lequel chaque canal de formation de faisceau comprend un transmetteur à basse tension comportant une sortie couplée au conducteur de signal de sonde pour le canal et un récepteur à basse tension comportant une entrée couplée au conducteur de signal de sonde pour le canal.

5. Système d'imagerie de diagnostic par ultrasons selon la revendication 4, dans lequel le conducteur de signal de sondes et le conducteur d'alimentation à haute tension sont contenus à l'intérieur d'un câble de la sonde ultrasonore.

6. Système d'imagerie de diagnostic par ultrasons selon la revendication 5, dans lequel la sonde ultrasonore est configurée avec une pluralité de canaux de sonde, chaque canal de sonde comportant un élément transducteur et étant couplé à un conducteur de signal de sonde respectif,
dans lequel un canal de sonde comprend en outre un commutateur de transmission et un transmetteur à haute tension couplés en série entre un conducteur de signal de sonde et un élément transducteur, et un commutateur de transmission/réception couplé en parallèle avec le transmetteur à haute tension.

7. Système d'imagerie de diagnostic par ultrasons selon la revendication 6, dans lequel le conducteur d'alimentation à haute tension est couplé à chacun des transmetteurs à haute tension.

8. Système d'imagerie de diagnostic par ultrasons selon la revendication 7, dans lequel chaque canal de sonde comprend en outre un second élément transducteur,
dans lequel chaque canal de sonde comprend en outre un second commutateur de transmission et un second transmetteur à haute tension couplés en série entre le conducteur de signal de sonde pour ce canal et le second élément transducteur pour ce canal et un second commutateur de transmission/réception couplé en parallèle avec le second transmetteur à haute tension.

9. Système d'imagerie de diagnostic par ultrasons selon la revendication 7, dans lequel chaque canal de sonde comprend une pluralité d'éléments transducteurs,
dans lequel un canal de sonde comprend en outre un pluralité de micro-canaux de formation de faisceau, et chaque micro-canal de formation de faisceau comprend un élément retardateur couplé au conducteur de signal de sonde pour ce canal de sonde, un commutateur de transmission et un transmetteur à haute tension couplés en série entre l'élément retardateur et un élément transducteur, et un préamplificateur et un commutateur de transmission/réception couplés en série l'un avec l'autre et en parallèle avec le transmetteur à haute tension.

10. Système d'imagerie de diagnostic par ultrasons, comprenant :
un macroordinateur de système à ultrasons comprenant :
une alimentation à haute tension (22) ;
une pluralité d'entrées/sorties frontales, chaque entrée/sortie étant couplée à la sortie d'un transmetteur à basse tension (26') produisant un signal de transmission à basse tension et l'entrée d'un préamplificateur ou d'un récepteur (28) ;
un élément de formation de faisceau couplé aux entrées/sorties frontales ;
un processeur de signal ; et
un affichage ; et
une sonde ultrasonore (10) comprenant :
un câble de sonde (14) comportant un conducteur d'alimentation (60) couplé à l'alimentation à haute tension et une pluralité de conducteurs de signal couplés aux entrées/sorties frontales ;
une pluralité d'éléments transducteurs (12') ;
une pluralité de transmetteurs à haute tension (16), chacun couplé au conducteur d'alimentation et chacun comportant une entrée couplée à un conducteur de signal pour recevoir le signal de transmission à basse tension et une sortie couplée à un élément transducteur ; et
une pluralité de commutateurs de transmission/réception (T/R), chacun couplé en parallèle avec un transmetteur à haute tension.

11. Système d'imagerie de diagnostic par ultrasons selon la revendication 10, dans lequel les transmetteurs à basse tension et préamplificateurs ou récepteurs du frontal de macroordinateur de système à ultrasons sont fabriqués sous forme de circuits intégrés à basse tension.

12. Système d'imagerie de diagnostic par ultrasons selon la revendication 10, dans lequel les transmetteurs à haute tension et les commutateurs de transmission/réception de la sonde ultrasonore sont fabriqués sous forme de circuits intégrés à haute tension.

13. Système d'imagerie de diagnostic par ultrasons selon la revendication 10, dans lequel l'alimentation fournit deux hautes tensions complémentaires,
dans lequel le câble de sonde comprend en outre des premier et second conducteurs d'alimentation pour les deux tensions complémentaires.

14. Système d'imagerie de diagnostic par ultrasons selon la revendication 10, dans lequel les transmetteurs à haute tension comprennent chacun un étage de sortie pulsé.

15. Système d'imagerie de diagnostic par ultrasons selon la revendication 10, dans lequel les transmetteurs à haute tension comprennent chacun un étage de sortie comprenant un amplificateur linéaire comportant une entrée et une sortie et étant alimenté par des hautes tensions complémentaires.
